# EUROPEAN PATENT SPECIFICATION

(11) **EP 2 180 912 B1**
(45) Date of publication and mention of the grant of the patent: **20.01.2021**
(21) Application number: 08776000.5
(22) Date of filing: 18.07.2008
(51) Int. Cl.: A61M 5/20, A61M 5/32

(54) **INJECTION SYSTEM WITH BASE STATION**
INJEKTIONSSYSTEM MIT BASISSTATION
SYSTÈME D'INJECTION MUNI D'UNE STATION DE BASE

(30) Priority: 29.08.2007 GB 0716774
(43) Date of publication of application: 05.05.2010
(73) Proprietor: Cilag GmbH International, 6300 Zug (CH)
(72) Inventor: JENNINGS, Douglas, Ivan, Royston Hertfordshire SG8 7XU (GB); BURNELL, Rosemary, Louise, Cambridge CB1 7TS (GB)
(74) Representative: Tunstall, Christopher Stephen
(86) International application number: PCT/GB2008/002475
(87) International publication number: WO 2009/027621

(56) References cited:
- EP-A- 1 518 575
- WO-A-2007/036676
- FR-A- 2 654 938
- US-A- 5 807 334
- US-A1- 2002 151 839
- US-A1- 2005 261 633
- US-A1- 2006 069 350
- US-A1- 2006 258 986

## Description

### Field of the Invention

The present invention relates to an injection system, particularly for use in conjunction with a reusable injection device which has a syringe and which extends the syringe, discharges its contents and then retracts it automatically after the contents are fully discharged.

### Background of the Invention

It is known to use automatic injection devices (also referred to as auto-injectors) for subcutaneous injections, particularly for self-delivery of pharmacological products by patients in the home. One known feature of an auto-injector is automatic injection by extending the needle of a syringe out of the body of the auto-injector, followed by delivery of the pharmacological product through the needle of the syringe and retraction of the syringe. It is very useful for a patient to be able to undertake a self-administrated injection without assistance from a medical practitioner, for example in emergency circumstances or on a regular basis in a treatment programme.

It is desirable that an auto-injector be easily and safely operable in order to ensure that a self-administrated injection is performed successfully. Known injection devices are described in WO 95/35126 and EP-A-0 516 473. These devices employ a drive spring and some form of release mechanism that release the syringe from the influence of the drive spring and allow it to be retracted by a return spring once its contents are supposed to have been discharged.
page la inserted here

The majority of present auto-injectors are disposable. However, it is expensive to provide a whole new auto-injector for every injection. The cost can be significantly reduced by making the injection device (or at least part of it) reusable. However, reusable auto-injectors also have some drawbacks, for example they may be complicated to reassemble and/or be dangerous for patients if any misuse occurs.

FR 2 654 938 A discloses an injection kit including a casing having a housing for receiving a syringe cartridge and a recess for receiving a syringe administering device. The syringe cartridge has a syringe with a needle and a sleeve assembly for the syringe. The syringe administering device has a trigger and a release mechanism operable to control a discharging means to discharge the substance in the syringe. In use, the syringe administering device is removed from the recess and connected with the syringe cartridge. The assembled device is then removed from the housing for injection. The syringe administering device is reusable and after an injection, the administering device is detached from the syringe cartridge and inserted into the recess. The recess includes means to reset the release mechanism when the device is placed into the recess.

US 2006/069350 A1 discloses an apparatus for holding a syringe and injecting fluid from the syringe. The apparatus includes a barrel having a bore extending therethrough for receiving a body portion of the syringe, a drive spring, and a telescoping piston movable under the influence of the drive spring to advance the syringe from a retracted position within the barrel to an advanced position wherein a needle portion of the syringe extends outwardly of the barrel, and to compress a plunger portion of the syringe relative to the body portion of the syringe to inject the fluid through the needle portion. After the fluid is injected, the needle is retracted back into a shielded position

US 2002/151839 A1 discloses a needle-less injector with an external resetting mechanism after usage of the injector.

US 2005/261633 A1 discloses a rechargeable handheld injection device that supports stock syringes for injection/removal of fluid or fat cells. The injection device comprises a housing, an adjustable/replaceable syringe cradle assembly, an electrical actuator with settings of dual speed that actuate the syringe cradle assembly in forward and reversible drive using forward and reverse buttons, a trigger switch to control the actuator, a preprogrammed syringe control panel, a needle guard assembly and a rechargeable battery in conjunction with a switch button and electronic component. A separate wall mount battery charger is used to charge the battery of the handheld device.

EP 1 518 575 A discloses an automatic administration instrument for injecting a drug solution filled in a syringe, wherein a switching means provided on the body of the administration instrument is operated by pressing a part of the exterior of the body against a body region of a patient to which the drug solution is to be administered, thereby operating a first driving means so that an injection needle housed in the body protrudes from the body to perform needle insertion into the body region, and thereafter, a second driving means for driving the syringe is operated to administer the drug solution.

### Summary of the Invention

The injection system of the present invention is designated to deal with the above mentioned problems.

The invention is defined according to claim 1.

The base station allows the injection device to be reset automatically without significant user intervention so that reliable reusability of the injection device is achieved. The injection device itself can be manufactured as two separate sub-assemblies: a first replaceable subassembly and a second reusable subassembly. The replaceable subassembly can be removed following operation of the device and replaced with a new replaceable subassembly when the injection device has been reset to its unactuated state. In one embodiment of the present invention, the actuator mechanism comprises a biasing element which in the unactuated state of the actuator mechanism is in a first unextended position and which in the actuated state of the actuator mechanism is in a second extended position. The base station may comprise an engaging mechanism for applying force to the biasing element to reset it from its extended position to its unextended position.

Preferably, an end of the biasing element is movable from a first position to a second position to move the syringe to its extended position. The engaging mechanism moves the said end of the biasing element from the second position to the first position to reset it.

Preferably, the engaging mechanism is an arm adapted to connect to the biasing element. The arm connects to the biasing element via an aperture in the housing. Preferably, the arm comprises a screw thread which is engageable with the drive of the injection device and the base station further comprises a motor connected to the arm to rotate the arm and return actuator mechanism to its first position and return the drive to a position in which it can be acted upon by the actuator mechanism to move the syringe to its extended position and expel its contents.

In a second embodiment of the present invention, the actuator mechanism comprises a motor to extend the syringe and expel the contents of the syringe.

Preferably, the actuator mechanism comprises a rechargeable element to power the motor. The rechargeable element may comprise a rechargeable capacitor. It may also comprise a rechargeable battery.

Preferably, the base station comprises a recharging unit adapted to recharge the rechargeable element when the injection device is connected to the base station. The base station may be powered by mains electricity. It may also be powered by a battery.

Advantageously, the injection device of the present invention is reusable. The syringe is replaceable. Other components which interact directly with the syringe may also be replaceable. These components form a replaceable assembly.

Moreover, the base station may be further adapted to reset the trigger mechanism so that the actuator mechanism in its unactuated state is prevented by the trigger mechanism from allowing the syringe to move to its extended position.

Preferably, the trigger mechanism and actuator mechanism are arranged in the injection device so that the trigger mechanism is reset when the actuator mechanism is reset.

Preferably, the injection device comprises a second biasing element adapted to reset the trigger mechanism when the actuator mechanism is reset.

A retraction element in the injection device may bias the syringe from its extended position to its retracted position after fluid has been expelled from the syringe. However, the actuator mechanism will still be in a position in which it has been actuated, even though the syringe has been moved to its retracted position. This means that the injection device cannot be reused by simply replacing the syringe.

Preferably, the injection device further comprises a decoupling mechanism, wherein the drive and actuator mechanism are decoupled from each other by the decoupling mechanism after fluid has been expelled so that the syringe can be moved from its extended position to its retracted position by the retraction element.

In a second aspect of the present invention, there is provided an injection device, comprising:
a syringe comprising a fluid container and a discharge nozzle;
a housing adapted to receive the syringe which is movable between a retracted position in which the syringe is wholly contained in the housing and an extended position in which at least part of discharge nozzle extends out of the housing;
a drive;
an actuator mechanism connected to the drive having an unactuated state in which it does not act on the drive and an actuated state in which it has acted on the drive and moved the syringe to its extended position and expelled fluid from the fluid container; and
a trigger mechanism which is operable to release the actuator mechanism to move the syringe to its extended position and expel fluid from the fluid container,
wherein the housing is adapted to connect with a base station and the actuator mechanism is adapted to be reset by the base station from its actuated state to its unactuated state so that further actuation of the actuator mechanism can take place.

In a third aspect of the present invention, there is provided a base station adapted to connect to the aforementioned injection device and reset the actuator mechanism from its actuated state to its unactuated state so that further actuation of the actuator mechanism can take place.

### Brief Description of the Drawings

The present invention will now be described below by way of reference to the accompanying drawings in which:
Fig. 1a is a right-side view of an injection device for use with an injection system according to a first embodiment of the present invention;
Fig. 1b is a perspective view of the injection device of Fig. 1 with its cap removed;
Fig. 1c is a perspective view of the cap of the injection device of Fig. 1;
Fig. 2a is an exploded right-side view of the injection device of Fig. 1;
Fig. 2b is a right-side view of the assembled components of the injection device of Fig.1;
Figs. 3a-3e are perspective views of the injection system according to a first embodiment of the present invention;
Fig. 4 is a perspective view of an injection device for use with an injection system according to a second embodiment of the present invention;
Fig. 5a is an exploded right-side view of the injection device of Fig. 4;
Fig. 5b is a right-side view of the injection device of Fig. 4;
Figs. 6a-6e are perspective views of the injection system according to the second embodiment of the present invention; and
Fig. 7 is a schematic view of the system of Figs. 6a-6e.

### Detailed Description of the Drawings

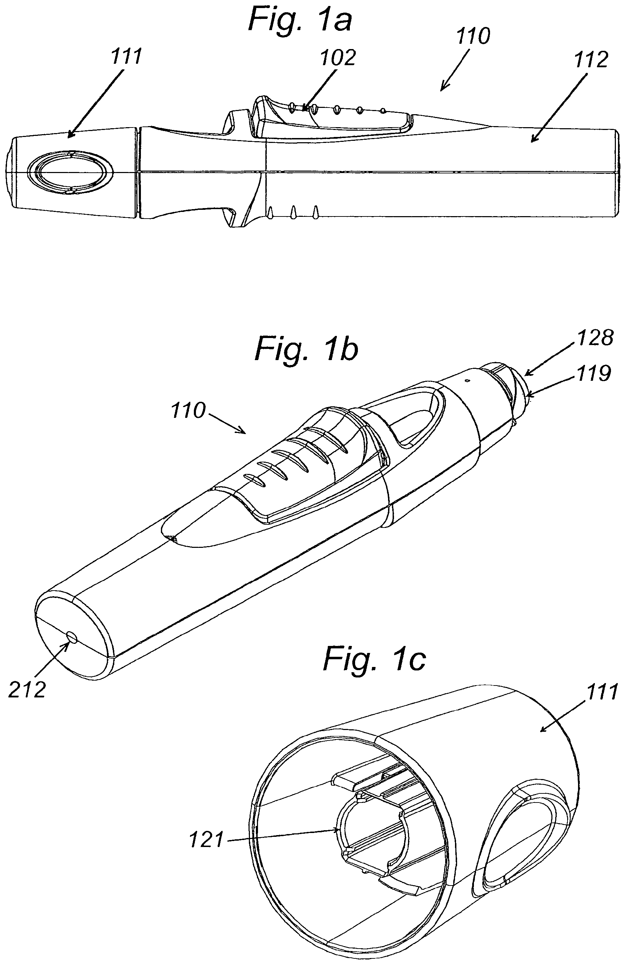

Fig. 1a is a right side view of an injection device 110 according to the present invention. The injection device 110 has a housing 112, a cap 111 which is removable from a proximal end 167 of the housing 112 and a trigger button 102. Other parts of the device will be described in greater detail below.

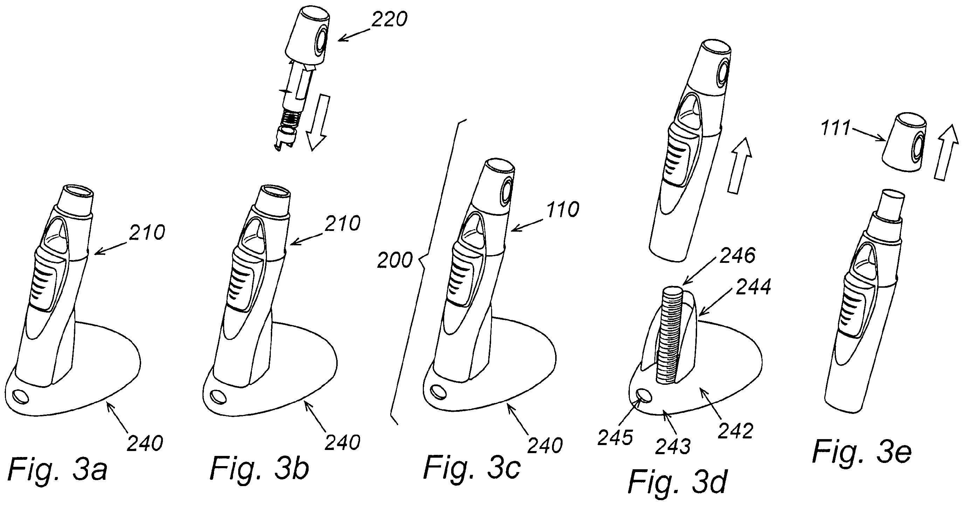

Fig. 1b is a perspective view of the injection device 110 according to the present invention with the cap (not shown) removed from its end. The end of the housing 112 has an exit aperture 128, from which the end of a sleeve 119 can be seen to emerge. There is also an aperture 212 in the housing 112, the function of which is explained in conjunction with Figs. 3a to 3e below.

Fig. 1c is a perspective view of the cap 111 of the injection device 110 according to the present invention. The cap 111 has a central boss 121 that fits within the sleeve 119 when the cap 111 is installed on the housing.

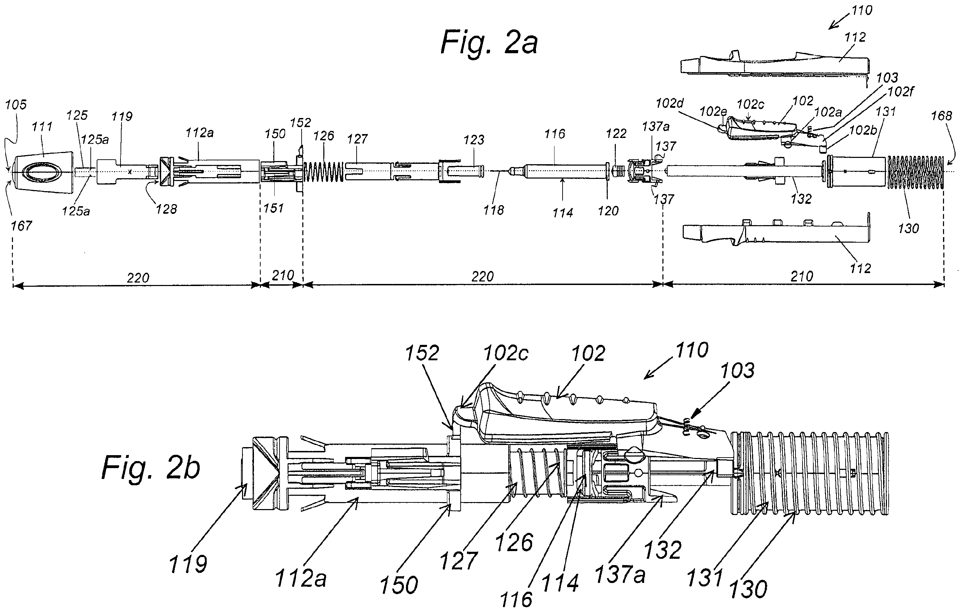

Fig. 2a is an exploded right-side view of the injection device 110 according to the present invention and Fig. 2b is a right-side view of the assembled components of the injection device 110 according to the present invention without the housing 112 or cap 111.

The injection device 110 comprises two sub-assemblies of components: a first reusable assembly 210 and a second replaceable assembly 220.

As illustrated, the injection device 110 comprises a hypodermic syringe 114 of conventional type, including a syringe body 116 terminating at one end in a discharge nozzle, specifically a hypodermic needle 118, and at the other in a flange 120. The conventional plunger that would normally be used to discharge the contents of the syringe 114 manually has been removed and replaced with a drive element (referred to below as the drive element 132) that contacts a bung 122 in the syringe 114. The bung 122 contains a drug (not shown) to be administered within the syringe body 116. Whilst the syringe illustrated is of hypodermic type, this need not necessarily be so. Transcutaneous or ballistic dermal and subcutaneous syringes may also be used with the injection device of the present invention.

As illustrated, the injection device 110 includes a return spring 126 that biases the syringe 114 from an extended position in which the needle 118 extends from the aperture 128 in a case nose 112a of the housing 112 to a retracted position in which the needle 118 is contained within the housing 112. The return spring 126 acts on the syringe 114 via a syringe carrier 127. The syringe 114 is moveable along a longitudinal axis 105 of the injection device 110 which extends centrally along the length of the injection device from the exit aperture 128 at its proximal end 167 to a distal end 168.

At the distal end 168 of the housing 112 is an actuator, which here takes the form of a compression drive spring 130. Drive from the drive spring 130 is transmitted via a drive element 132 to the syringe 114 to advance it from its retracted position to its extended position and discharge its contents through the needle 118. The drive accomplished this task by acting directly on the drug and the syringe 114. Hydrostatic forces acting through the drug and, to a lesser extent, static friction between the bung 122 and the syringe body 116 initially ensure that they advance together, until the return spring 126 bottoms out on the syringe carrier 127 or meets some other obstruction (not shown) that retards its motion.

A drive sleeve 131 takes drive from the drive spring 130 and transmits it to a delay piston 133 on a drive element 132. The drive element 132 includes a hollow stem, the inside of which can be accessed through aperture 212.

The trigger button 102 is provided on the side of the housing 112 which, when in an engaged position with a proximal end 145 of the drive sleeve 131, serves to retain the drive spring 130 in a compressed state by contact between the locking surface 102b and the drive sleeve 131 when the button 102 is in a deactivated position. The trigger button 102 can pivot on the housing 112 via pivot 102a. When downwards pressure is applied to the trigger button 102 at an activation surface 102c (i.e. pressure directed into the housing 112), the locking surface 102b moves upwards in a direction out of the housing 112. In this activated position of the button 102, the locking surface 102b is decoupled from the drive sleeve 131, thereby allowing the drive sleeve 131 to move relative to the housing 112 towards the exit aperture 128 under the influence of the drive spring 130.

The sliding sleeve 119 is moveable from its extended position (as shown in Fig. 1b) where it protrudes out of the exit aperture 128 into a retracted position in the case nose 112a of the housing 112. The sliding sleeve 119 is connected to a button lock element 150 which has resilient arms 151 which bias the sliding sleeve 119 into its extended position in which its end protrudes from the end of the case nose 112a. Thus, application of pressure to the end of the sliding sleeve 119, for example by pressing the end of the sliding sleeve 19 against tissue, causes it to move into its retracted position into the housing 112; release of the pressure causes the sliding sleeve 119 to move into its extended position under bias from the resilient arms 151 acting against a side wall the housing 112. The button lock element 150 has a button lock protrusion 152 which contacts with the end of a trigger button protrusion 102d on the trigger button 102 when the sliding sleeve is in its extended position. The trigger button protrusion 102d extends in a direction which is generally parallel to the longitudinal axis 105 of the injection device 110. The button lock protrusion 152 extends in a direction which is generally perpendicular to the longitudinal axis 105 towards the trigger button protrusion 102d. The trigger button protrusion 102d has an aperture 102e which can move over the button lock protrusion 152 when the button lock element 150 has been moved away from the exit aperture 128 (i.e. when the sliding sleeve 119 has been moved into the exit aperture 128 into its retracted position). In this position, the trigger button 102 can be moved into its activated position by rotating the trigger button 102 about the pivot 102a in the direction of the pressure applied to the pressure surface 102c. Thus, the button lock element 150 and the sliding sleeve 119 act together to lock the trigger button 102 in its activated position (i.e. the locking surface 102b contacts the end of the drive sleeve 131 preventing it from moving towards the exit aperture under the bias of the compressed drive spring 130).

When the sliding sleeve 119 has been moved into its retracted position (i.e. unlocked position) and the trigger button 102 has been rotated into its activated position, the operation of the device is then as follows.

Initially, the drive spring 130 moves the drive sleeve 131 and the drive sleeve 131 moves the drive element 132. The drive element 132 moves and, by virtue of static friction and hydrostatic forces acting through the drug (not shown), moves the syringe body 116 and the syringe carrier 127 against the action of the return spring 126. The return spring 126 compresses and the hypodermic needle 118 emerges from the exit aperture 128 of the housing 112. This continues until the return spring 126 bottoms out or the syringe body 116 meets some other obstructions (not shown) that retards its motion. Because the static friction between the drive element 132 and the syringe body 116 and the hydrostatic forces acting through the drug (not shown) to be administrated are not sufficient to resist the full drive force developed by the drive spring 130, at this point the drive element 132 begins to move within the syringe body 116 and the drug (not shown) begins to be discharged. Dynamic friction between the drive element 132 and the syringe body 116 and the hydrostatic forces acting through the drug (not shown) to be administrated are, however, sufficient to retain the return spring 126 in its compressed state, so the hypodermic needle 118 remains extended.

After a time, the drive element 132 completes its travel within the syringe body 116 and can go no further. At this point, the contents of the syringe 114 are completely discharged and the force exerted by the drive spring 130 acts to retain the drive element 132 in its terminal position. Flexible latch arms 132a linking the drive sleeve 131 with the drive element 132 reach a constriction (not shown) in the housing 112. This constriction moves the flexible latch arms 132a inwards from the position shown to a position at which they no longer couple the drive sleeve 131 to the drive element 132, aided by bevelled surfaces on the constriction. Once this happens, the drive sleeve 131 acts no longer on the drive element 132, allowing them to move relative to each other. At this point, of course, the syringe 114 is released, because the forces developed by the drive spring 130 are no longer being transmitted to the syringe 114, and the only force acting on the syringe will be the return force from the return spring 126. Thus, the syringe 114 is now returned to its retracted position and the injection cycle is complete.

All this takes place, of course, only once the cap 111 has been removed from the end of the housing 112. The end of the syringe is sealed with a boot 123. The central boss 121 of the cap that fits within the sleeve 119 when the cap 111 is installed on the housing 112 comprises a retainer element 125 which is fixed into the boss 121. The retainer element 125 comprises resilient protrusions 125a which are directed away from the exit aperture 128. These resilient protrusions 125a deform as the cap 111 is inserted onto the housing 112 over the boot 123. The protrusions 125a then grip the boot 123 tightly so that the ends of the protrusions are slightly embedded in the boot 123 which might be made from rubber. This means that, as the cap 111 is pulled off the housing 112, the boot 123 is pulled away from the syringe 114 with the cap 111.

Fig. 2a also shows a syringe lock protrusion 170 located on the button 102 at its distal end which is proximal to the end which is located nearest to the aperture 128. The syringe lock protrusion 170 extends in a generally perpendicular direction (with respect to the longitudinal axis 105) into the injection device 110 towards the longitudinal axis 105.

The first reusable assembly 210 comprises the following components: the housing 112, the drive element 132, the drive sleeve 131, the drive spring 130, the trigger button 102 and the button lock element 150.

The second replaceable assembly 220 comprises the following components: the cap 111, the needle shield 123, the shield retainer 125, the syringe 114, the case nose 112a, the syringe carrier 127, the sliding sleeve 119, the latch actuator element 137a connected to the end of the syringe carrier 127 and the return spring 126. The replaceable assembly 220 is intended to contain all of the drug and sharp components of the injection device, so that any possible danger or damage caused by an adventitious misuse or inappropriate disposal of the injection device is reduced.

Figs. 3a-3e are perspective views of the injection system according to a first embodiment of the present invention. They illustrate the operation of a reusable injection system 200 of the present invention which comprises a base station 240 and the injection device 110 as described above. As stated above, the injection device 110 includes the reusable assembly 210 and the replaceable assembly 220.

The base station 240 comprises a base housing 242 having a plane surface 243 enabling the base station to sit on a horizontal surface, such as a table; a support 244 protruding at a slight angle (e.g. an angle in the range of 0 to 20 degrees) from an axis which is perpendicular to the plane surface 243; and a switch 245 which is able to be switched on and off to control resetting of the injection device 110. The support 244 is shaped and adapted to support the injection device 110 when the injection device 110 is connected to the base station 240.

The connection arrangement between the injection device 110 and the base station 240 of the present invention is shown in Fig. 3d. A threaded arm 246 protrudes from the base station 240 in a direction which is generally parallel to the support 244. The arm 246 has a distal end 246a which is spaced from the base station 240 and a proximal end (not shown) which is connected to a motor (not shown) contained in the housing of the base station 242. The arm's distal end 246a is adapted to connect to the drive element 132 of the injection device 110 through an aperture 212 at the distal end 168 of the housing of the injection device 112 when the injection device 110 is placed onto the base station 240 and supported by the support 244. In this arrangement, the arm engages with a releasable threaded fixture (not shown) contained in the hollow drive sleeve 132 and fixed to the drive sleeve 132. The arm 246 is able to reset the injection device 110 from its actuated state to its unactuated state by rotating under force of the motor through the releasable threaded fixture causing the entire drive sleeve 132 to move to the distal end 168 of the housing 112. As the drive sleeve 132 moves towards the distal end 168 of housing 112, compressible wings 134 on opposing sides of the drive element 132 connect with flange-like elements (not shown) contained within the hollow drive sleeve 132, thereby returning the drive element 132, drive sleeve 131 and the drive spring 130 to their initial unactuated state. In this state, flexible latch arms (not shown) on the drive sleeve 132 reengage with corresponding protrusions (not shown) on the drive sleeve 131, thereby locking the drive sleeve 132 to the drive element 132.

In alternative embodiments of the present invention, the threaded arm 246 could be replaced with any other means such as a lead screw, rack and pinion, and pulley.

The trigger button 102 also becomes reset under a resilient force from a button return spring 103 mounted on the distal end surface 102f of the trigger button 102. This resilient force is in a direction which is generally perpendicular to the longitudinal axis 105 of the injection device towards the housing 112. During actuation of the trigger button 102, when the pressure is applied to the pressure surface 102c of the trigger button 102 so that the button 102 rotates into its activated position, the button return spring 103 is compressed and biases against the housing 112.

As the drive spring 130, drive sleeve 131 and drive element 132 are reset, the trigger button 102 is allowed to rotate when the proximal end of drive sleeve 131 passes the locking protrusion 102b, so that the button return spring 103 returns the trigger button 102 to its deactivated position and the drive sleeve 131 is locked against the locking protrusion 102b.

Fig. 3a is a perspective view of the injection system 200 of the present invention, after actuation of the device and its placement on the base station 240. The base station 240 is resetting the injection device 110. After an injection operation, the replaceable assembly 220 of the injection device 110 are disposed of, so that all fluid contaminated parts of the injection device 110 are removed. Then a reusable assembly 210 of the injection system 110 is placed onto the base station 240 and against the support 244. When the switch 245 is switched on, the reset occurs as explained above.

Fig. 3b is a perspective view of the injection system 200 of the present invention when a new replaceable assembly 220 of the injection device 110 is about to be inserted into the housing 112 of the reusable assembly 210 of the injection device 110.

Fig. 3c is a perspective view of the injection system 200 of the present invention with the whole injection device 110 placed onto the base station 240 after the new replaceable assembly 220 has been inserted into the housing 112 of the reusable assembly 210 of the injection device 110.

Fig. 3d is a perspective view of the injection system 200 of the present invention with the whole injection device 110 removed from the base station 240.

Fig. 3e is a perspective view of the injection device of the present invention when the cap 111 is removed from the injection device 110, so that an injection can take place.

A second embodiment of the injection system of the present invention uses an electric motor to actuate the injection. The motor has a second function of resetting the actuator after an injection operation. The electric power to drive the motor is provided by a rechargeable element. The base station is used to recharge the rechargeable element in the injection device.

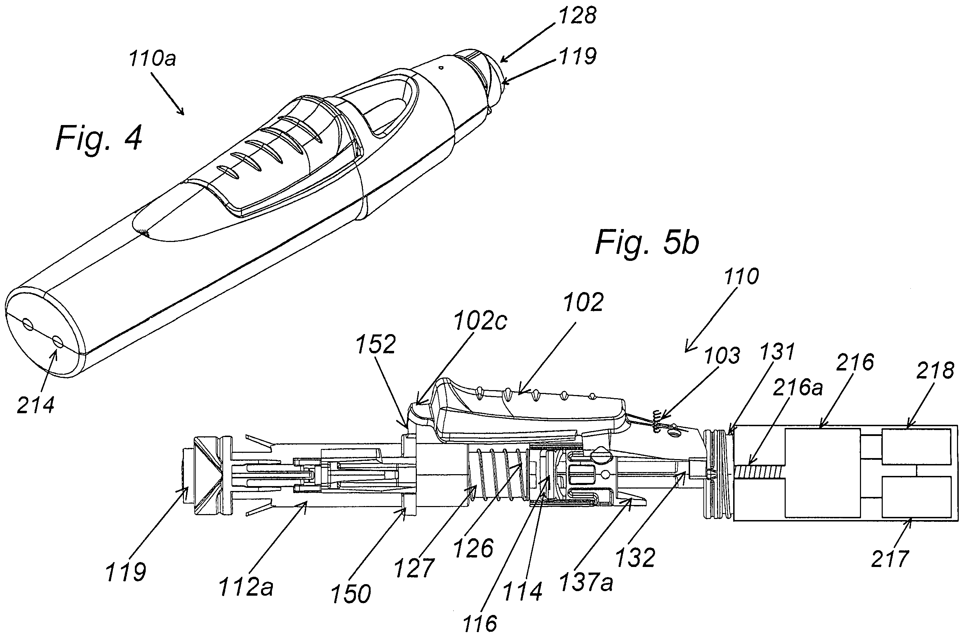

Fig. 4 is a perspective view of an alternative electrically-operated injection device 110a according to the second embodiment of the present invention. When the injection device 110a is connected to the base station 240a and the switch 245 is operated, recharging of the electrically operated injection device 110 occurs.

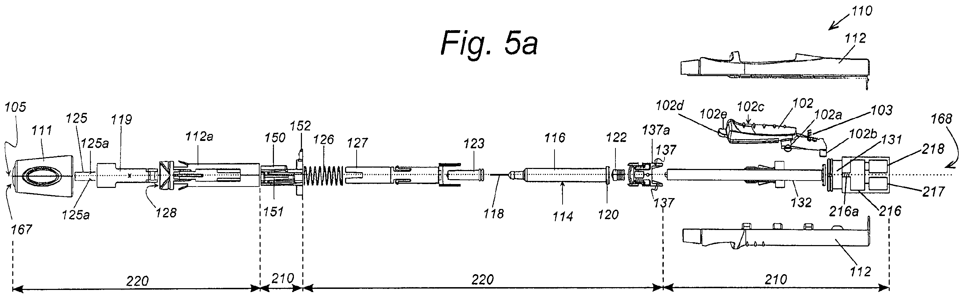

Figs. 5a and 5b are right-side views of the injection device 110a of Fig. 4. The drive element 132 is coupled with an electric motor 216 via a threaded drive shaft 216a of the electric motor. The motor 216 is configured to drive the drive element 132 by rotating the threaded drive shaft 216a in the hollow drive element 132 which contains a threaded element (not shown). In this way, the drive element 132 moves towards the proximal end of the housing 167 and thereby moves the syringe 114 to fulfil an injection operation.

Reset of the injection device 110a can be achieved by controlling the motor 216 to rotate the threaded drive shaft 216a in an opposite direction to return the drive element 132 and drive sleeve 131 to its initial unactuated state (as outline above in respect of the first embodiment). This may be controlled by a motor control unit 217. For instance, after the drug in the syringe 114 is fully discharged and the injection device 110a is place on the base station 240a, the motor control unit 217 is signalled and controls the motor 216 to rotate the threaded drive shaft 216a in an opposite direction. The motor control unit 217 can reverse the direction of the electric current passing through the motor 216 and therefore alter the rotation direction of the drive shaft 216a.

The motor 216 is powered by a rechargeable battery 218a or alternatively another type of rechargeable unit, for example a rechargeable capacitor.

In the second embodiment of the present invention, the trigger button 102 acts as a switch connected to the control unit 217. Once the trigger button 102 is activated, the power to the motor 216 is turned on so that the drive element 132 is actuated and drives the syringe 114 to fulfil an injection.

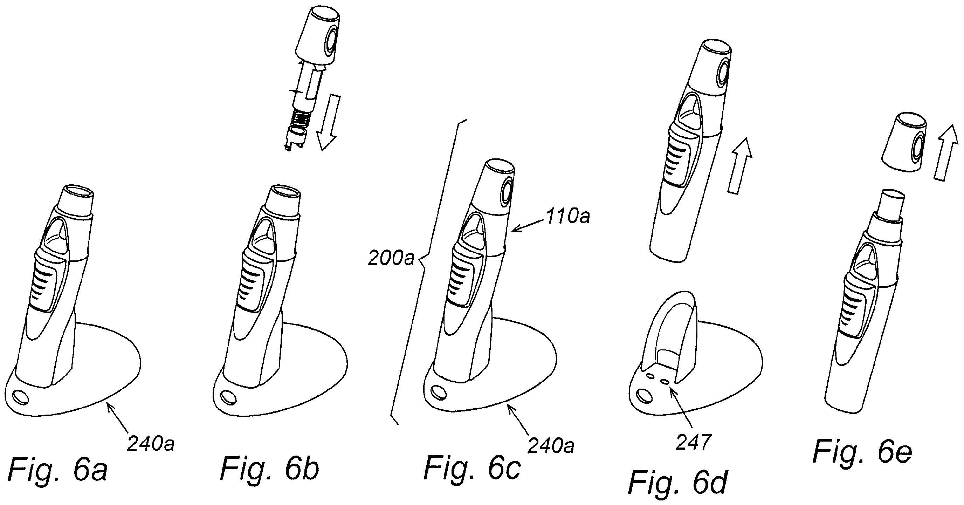

Figs. 6a-6e are perspective views of the injection system 200a according to the second embodiment of the present invention. Different from the injection device of Fig. 3, the motor-actuated electric injection device 110a replaces the drive spring actuated mechanical injection device 110, and the connection between the base station 240a and the injection device 110a is configured for recharging the rechargeable battery 218a.

Two electric contact elements 214 of the rechargeable element 218 are located at the distal end of the housing 112. Two contact elements 247 of the charger 248 of the base station 240a provide a charging interface between the base station 240a and the injection device 110a to recharge the rechargeable element 218 of the injection device 110a, as shown in Fig. 6d. The motor control unit 217 detects the application of current through the contact elements 247 and controls the motor 216 to rotate the threaded drive shaft 216a to reset the drive element 132, drive sleeve 131 and trigger button 102.

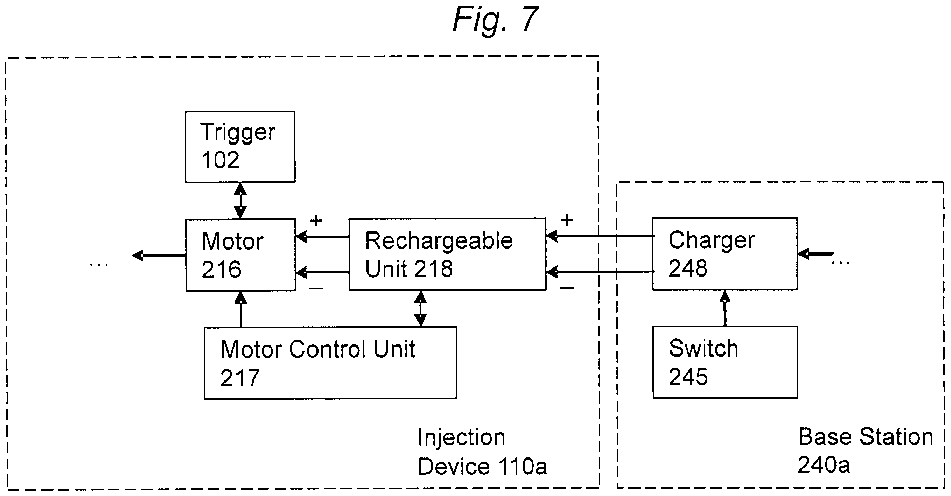

Fig. 7 is a schematic block diagram of the system of Figs. 6a-6e. The charger 248 in the base station 240a recharges the rechargeable unit 218 of the electric injection device 110a after the injection device 110a is placed on the base station 240a and the switch 245 is switched on. The rechargeable unit 218 of the injection device 110a powers the motor 216 to move the drive element 132 to extend the syringe 114 towards the proximal end 167 of the housing 112 for fulfilling an injection. The control circuit 217 is configured to detect current flowing into the injection device 110a from the charger 248 and operate the motor 216 to reset the drive sleeve 131, drive element 132 and trigger button 102 to their unactuated state.

The base station 240, 240a can be powered by either mains electricity or batteries to drive the motor in the base station 240 which is configured to reset the mechanical actuator according to the first embodiment of the present invention, and/or supply the charger 248 in the base station 240a according to the second embodiment of the present invention to charge the rechargeable unit 218 of the injection device 110a.

Thus, by providing a base station 240, 240a to reset the injection device 110, 110a and replacing the fluid contaminated parts of the injection device 220 after every injection operation while reusing a reusable fluid delivery assembly 210, the target of minimizing the amount of disposal to reduce the cost is achieved.

It will of course be understood that the present invention has been described above purely by way of example and modifications of detail can be made within the scope of the invention.

## Claims

1. An injection system comprising:
an injection device (110), the injection device comprising:
a replaceable syringe (114) comprising a fluid container (116) and a discharge nozzle (118);
a housing (112) adapted to receive the syringe which is movable between a retracted position in which the syringe is wholly contained in the housing and an extended position in which at least part of the discharge nozzle extends out of the proximal end of the housing;
a drive (132);
an actuator mechanism connected to the drive, the actuator mechanism having an unactuated state in which it does not act on the drive and an actuated state in which it has acted on the drive and moved the syringe to its extended position and expelled the contents of the fluid container; and
a trigger mechanism (102) which is operable to cause the actuator mechanism to move from its unactuated state to its actuated state and thereby move the syringe to its extended position and expel the contents of the fluid container; and
a base station (240);
wherein the injection device and the base station are adapted such that the base station connects to the distal end of the housing of the injection device and resets the actuator mechanism from its actuated state to its unactuated state so that further actuation of the actuator mechanism can take place.

2. The injection system according to claim 1, wherein the actuator mechanism comprises a motor (216) connected to the drive to extend the syringe and expel its contents.

3. The injection system according to claim 2, wherein the actuator mechanism comprises a rechargeable element (218) connected to the motor to power it.

4. The injection system according to claim 3, wherein the rechargeable element comprises a capacitor.

5. The injection system according to claim 3, wherein the rechargeable element comprises a rechargeable battery (218a).

6. The injection system according to any one of claims 3 to 5, wherein the base station comprises a recharging unit (248) adapted to recharge the rechargeable element when the injection device is connected to the base station.

7. The injection system of any one of claims 2 to 6, wherein, when the injection device is connected to the base station, the motor is controlled to return the drive to a position in which it can be acted upon by the actuator mechanism to move the syringe to its extended position.

8. The injection system according to claim 1, wherein the actuator mechanism comprises a biasing element (130) which in the unactuated state of the actuator mechanism is in a first unextended position and which in the actuated state of the actuator mechanism is in a second extended position.

9. The injection system according to claim 8, wherein the base station comprises an engaging mechanism adapted to apply force to the biasing element to reset it from its extended position to its unextended position.

10. The injection system according to claim 9, wherein the engaging mechanism moves the end of the biasing element from the second position to the first position to reset it.

11. The injection system according to claim 10, wherein the engaging mechanism is an arm (246) adapted to connect to the biasing element.

12. The injection system according to claim 11, wherein the arm connects to the biasing element via an aperture (212) in the housing.

13. The injection system of claim 12, wherein the arm comprises a screw thread which is engageable with the drive of the injection device and the base station further comprises a motor connected to the arm to rotate the arm and return actuator mechanism to its first position and return the drive to a position in which it can be acted upon by the actuator mechanism to move the syringe to its extended position and expel its contents.

14. The injection system according to any one of claims 1 to 13, wherein the base station is powered by mains electricity.

15. The injection system according to any one of claims 1 to 14, wherein the base station is powered by a battery.

16. The injection system according to any of claims 1 to 15, wherein the injection device is reusable.

17. The injection system according to any one of claims 1 to 16, wherein the base station is further adapted to reset the trigger mechanism so that the actuator mechanism in its unactuated state is prevented by the trigger mechanism from allowing the syringe to move to its extended position.

18. The injection system according to claim 17, wherein the trigger mechanism and actuator mechanism are arranged in the injection device so that the trigger mechanism is reset when the actuator mechanism is reset.

19. The injection system according to claim 18, wherein the injection device comprises a biasing component (103) adapted to reset the trigger mechanism when the actuator mechanism is reset.

20. The injection system according to any one of claims 1 to 19, wherein the injection device comprises a retraction element (126) adapted to bias the syringe from its extended position to its retracted position after the contents of the syringe have been expelled.

21. The injection system of claim 20, wherein the injection device further comprises a decoupling mechanism, wherein the drive and actuator mechanism are decoupled from each other by the decoupling mechanism after the contents of the syringe have been expelled so that the syringe can be moved from its extended position to its retracted position by the retraction element.

## Patentansprüche

1. Injektionssystem, umfassend:
eine Injektionsvorrichtung (110), wobei die Injektionsvorrichtung Folgendes umfasst:
eine ersetzbare Spritze (114), die einen Fluidbehälter (116) und eine Austragdüse (118) umfasst, ein Gehäuse (112), das zur Aufnahme der Spritze ausgeführt ist, die zwischen einer zurückgezogenen Position, in der die Spritze vollständig in dem Gehäuse enthalten ist, und einer ausgefahrenen Position, in der sich mindestens ein Teil der Austragdüse aus dem proximalen Ende des Gehäuses herauserstreckt, beweglich ist,
einen Antrieb (132),
einen mit dem Antrieb verbundenen Aktuatormechanismus, wobei der Aktuatormechanismus einen nicht betätigten Zustand, in dem er den Antrieb nicht beaufschlagt, und einen betätigten Zustand hat, in dem er den Antrieb beaufschlagt und die Spritze in ihre ausgefahrene Position bewegt und den Inhalt des Fluidbehälters ausgestoßen hat, und
einen Auslösermechanismus (102), der dahingehend betätigbar ist, den Aktuatormechanismus dazu zu veranlassen, sich aus seinem nicht betätigten Zustand in seinen betätigten Zustand zu bewegen und dadurch die Spritze in ihre ausgefahrene Position zu bewegen und den Inhalt des Fluidbehälters auszustoßen, und
eine Basisstation (240),
wobei die Injektionsvorrichtung und die Basisstation dazu ausgeführt sind, dass die Basisstation mit dem distalen Ende des Gehäuses der Injektionsvorrichtung verbunden wird und den Aktuatormechanismus aus seinem betätigten Zustand in seinen nicht betätigten Zustand rücksetzt, so dass eine weitere Betätigung des Aktuatormechanismus erfolgen kann.

2. Injektionssystem nach Anspruch 1, wobei der Aktuatormechanismus einen Motor (216) umfasst, der mit der Vorrichtung verbunden ist, um die Spritze auszufahren und ihren Inhalt auszustoßen.

3. Injektionssystem nach Anspruch 2, wobei der Aktuatormechanismus ein wiederaufladbares Element (218) umfasst, das mit dem Motor verbunden ist, um ihn mit Energie zu versorgen.

4. Injektionssystem nach Anspruch 3, wobei das wiederaufladbare Element einen Kondensator umfasst.

5. Injektionssystem nach Anspruch 3, wobei das wiederaufladbare Element eine wiederaufladbare Batterie (218a) umfasst.

6. Injektionssystem nach einem der Ansprüche 3 bis 5, wobei die Basisstation eine Ladeeinheit (248) umfasst, die dazu ausgeführt ist, das wiederaufladbare Element wieder aufzuladen, wenn die Injektionsvorrichtung mit der Basisstation verbunden ist.

7. Injektionssystem nach einem der Ansprüche 2 bis 6, wobei der Motor so gesteuert wird, wenn die Injektionsvorrichtung mit der Basisstation verbunden ist, dass er den Antrieb in eine Position rücksetzt, in der er durch den Aktuatormechanismus beaufschlagt werden kann, um die Spritze in ihre ausgefahrene Position zu bewegen.

8. Injektionssystem nach Anspruch 1, wobei der Aktuatormechanismus ein Vorspannelement (130) umfasst, das in dem nicht betätigten Zustand des Aktuatormechanismus in einer ersten, unausgefederten Position ist und das in dem betätigten Zustand des Aktuatormechanismus in einer zweiten, ausgefederten Position ist.

9. Injektionssystem nach Anspruch 8, wobei die Basisstation einen Eingriffsmechanismus umfasst, der dazu ausgeführt ist, das Vorspannelement mit Kraft zu beaufschlagen, um es aus seiner ausgefederten Position in seine unausgefederte Position rückzusetzen.

10. Injektionssystem nach Anspruch 9, wobei der Eingriffsmechanismus das Ende des Vorspannelements aus der zweiten Position in die erste Position bewegt, um es rückzusetzen.

11. Injektionssystem nach Anspruch 10, wobei der Eingriffsmechanismus ein Arm (246) ist, der dazu ausgeführt ist, mit dem Vorspannelement verbunden zu werden.

12. Injektionssystem nach Anspruch 11, wobei der Arm über eine Öffnung (212) in dem Gehäuse mit dem Vorspannelement verbunden wird.

13. Injektionssystem nach Anspruch 12, wobei der Arm ein Schraubengewinde umfasst, das mit dem Antrieb der Injektionsvorrichtung in Eingriff bringbar ist, und die Basisstation ferner einen mit dem Arm verbundenen Motor umfasst, um den Arm zu drehen und den Aktuatormechanismus in seine erste Position rückzusetzen und den Antrieb in eine Position rückzusetzen, in der er durch den Aktuatormechanismus beaufschlagt werden kann, um die Spritze in ihre ausgefahrene Position zu bewegen und ihren Inhalt auszustoßen.

14. Injektionssystem nach einem der Ansprüche 1 bis 13, wobei die Basisstation netzstromgespeist ist.

15. Injektionssystem nach einem der Ansprüche 1 bis 14, wobei die Basisstation batteriebetrieben ist.

16. Injektionssystem nach einem der Ansprüche 1 bis 15, wobei die Injektionsvorrichtung wiederverwendbar ist.

17. Injektionssystem nach einem der Ansprüche 1 bis 16, wobei die Basisstation ferner dazu ausgeführt ist, den Auslösermechanismus rückzusetzen, so dass der Aktuatormechanismus in seinem nicht betätigten Zustand durch den Auslösermechanismus daran gehindert wird, der Spritze zu gestatten, dass sie sich in ihre ausgefahrene Position bewegt.

18. Injektionssystem nach Anspruch 17, wobei der Auslösermechanismus und der Aktuatormechanismus in der Injektionsvorrichtung angeordnet sind, so dass der Auslösermechanismus rückgesetzt wird, wenn der Aktuatormechanismus rückgesetzt wird.

19. Injektionssystem nach Anspruch 18, wobei die Injektionsvorrichtung eine Vorspannkomponente (103) umfasst, die dazu ausgeführt ist, den Auslösermechanismus rückzusetzen, wenn der Aktuatormechanismus rückgesetzt wird.

20. Injektionssystem nach einem der Ansprüche 1 bis 19, wobei die Injektionsvorrichtung ein Rückzugselement (126) umfasst, das dazu ausgeführt ist, die Spritze aus ihrer ausgefahrenen Position in ihre zurückgezogene Position vorzuspannen, nachdem der Inhalt der Spritze ausgestoßen worden ist.

21. Injektionssystem nach Anspruch 20, wobei die Injektionsvorrichtung ferner einen Entkoppelmechanismus umfasst, wobei der Antrieb und der Aktuatormechanismus durch den Entkoppelmechanismus voneinander entkoppelt werden, nachdem der Inhalt der Spritze ausgestoßen worden ist, so dass die Spritze durch das Rückzugselement aus ihrer ausgefahrenen Position in ihre zurückgezogene Position bewegt werden kann.

## Revendications

1. Système d'injection, comprenant :
un dispositif d'injection (110), le dispositif d'injection comprenant :
une seringue remplaçable (114) comprenant un récipient de fluide (116) et une buse de décharge (118) ;
un boîtier (112) prévu pour recevoir la seringue, qui peut être déplacé entre une position rentrée dans laquelle la seringue est contenue entièrement dans le boîtier et une position sortie dans laquelle au moins une partie de la buse de décharge s'étend hors de l'extrémité proximale du boîtier ;
un dispositif d'entraînement (132) ;
un mécanisme d'actionnement connecté au dispositif d'entraînement, le mécanisme d'actionnement ayant un état non actionné dans lequel il n'agit pas sur le dispositif d'entraînement et un état actionné dans lequel il a agi sur le dispositif d'entraînement et a déplacé la seringue dans sa position sortie et a expulsé le contenu du récipient de fluide ; et
un mécanisme de déclenchement (102) qui peut être actionné pour provoquer le déplacement du mécanisme d'actionnement de son état non actionné à son état actionné et par conséquent le déplacement de la seringue dans sa position sortie et l'expulsion du contenu du récipient de fluide ; et
une station de base (240) ;
le dispositif d'injection et la station de base étant prévus de telle sorte que la station de base se raccorde à l'extrémité distale du boîtier du dispositif d'injection et réinitialise le mécanisme d'actionnement de son état actionné à son état non actionné de telle sorte qu'un actionnement supplémentaire du mécanisme d'actionnement puisse avoir lieu.

2. Système d'injection selon la revendication 1, dans lequel le mécanisme d'actionnement comprend un moteur (216) connecté au dispositif d'entraînement pour faire sortir la seringue et expulser son contenu.

3. Système d'injection selon la revendication 2, dans lequel le mécanisme d'actionnement comprend un élément rechargeable (218) connecté au moteur de manière à l'alimenter en puissance.

4. Système d'injection selon la revendication 3, dans lequel l'élément rechargeable comprend un condensateur.

5. Système d'injection selon la revendication 3, dans lequel l'élément rechargeable comprend une batterie rechargeable (218a).

6. Système d'injection selon l'une quelconque des revendications 3 à 5, dans lequel la station de base comprend une unité de recharge (248) prévue pour recharger l'élément rechargeable lorsque le dispositif d'injection est connecté à la station de base.

7. Système d'injection selon l'une quelconque des revendications 2 à 6, dans lequel, lorsque le dispositif d'injection est connecté à la station de base, le moteur est commandé de manière à ramener le dispositif d'entraînement dans une position dans laquelle le mécanisme d'actionnement peut agir sur celui-ci pour déplacer la seringue dans sa position sortie.

8. Système d'injection selon la revendication 1, dans lequel le mécanisme d'actionnement comprend un élément de sollicitation (130) qui, dans l'état non actionné du mécanisme d'actionnement, est dans une première position non sortie et qui, dans l'état actionné du mécanisme d'actionnement, est dans une deuxième position sortie.

9. Système d'injection selon la revendication 8, dans lequel la station de base comprend un mécanisme d'engagement prévu pour appliquer une force à l'élément de sollicitation pour le réinitialiser de sa position sortie à sa position non sortie.

10. Système d'injection selon la revendication 9, dans lequel le mécanisme d'engagement déplace l'extrémité de l'élément de sollicitation de la deuxième position à la première position pour le réinitialiser.

11. Système d'injection selon la revendication 10, dans lequel le mécanisme d'engagement est un bras (246) prévu pour se connecter à l'élément de sollicitation.

12. Système d'injection selon la revendication 11, dans lequel le bras se raccorde à l'élément de sollicitation par le biais d'une ouverture (212) dans le boîtier.

13. Système d'injection selon la revendication 12, dans lequel le bras comprend un filetage de vis qui peut être mis en prise avec le dispositif d'entraînement du dispositif d'injection et la station de base comprend en outre un moteur connecté au bras pour faire tourner le bras et ramener le mécanisme d'actionnement dans sa première position et ramener le dispositif d'entraînement à une position dans laquelle le mécanisme d'actionnement peut agir sur celui-ci pour déplacer la seringue dans sa position sortie et expulser son contenu.

14. Système d'injection selon l'une quelconque des revendications 1 à 13, dans lequel la station de base est alimentée en puissance par l'électricité du secteur.

15. Système d'injection selon l'une quelconque des revendications 1 à 14, dans lequel la station de base est alimentée en puissance par une batterie.

16. Système d'injection selon l'une quelconque des revendications 1 à 15, dans lequel le dispositif d'injection est réutilisable.

17. Système d'injection selon l'une quelconque des revendications 1 à 16, dans lequel la station de base est en outre prévue pour réinitialiser le mécanisme de déclenchement de telle sorte que dans l'état non actionné du mécanisme d'actionnement, le mécanisme de déclenchement empêche le mécanisme d'actionnement de permettre le déplacement de la seringue dans sa position sortie.

18. Système d'injection selon la revendication 17, dans lequel le mécanisme de déclenchement et le mécanisme d'actionnement sont agencés dans le dispositif d'injection de telle sorte que le mécanisme de déclenchement soit réinitialisé lorsque le mécanisme d'actionnement est réinitialisé.

19. Système d'injection selon la revendication 18, dans lequel le dispositif d'injection comprend un composant de sollicitation (103) prévu pour réinitialiser le mécanisme de déclenchement lorsque le mécanisme d'actionnement est réinitialisé.

20. Système d'injection selon l'une quelconque des revendications 1 à 19, dans lequel le dispositif d'injection comprend un élément de rétraction (126) prévu pour solliciter la seringue de sa position sortie à sa position rentrée après que le contenu de la seringue a été expulsé.

21. Système d'injection selon la revendication 28, dans lequel le dispositif d'injection comprend en outre un mécanisme de désaccouplement, le dispositif d'entraînement et le mécanisme d'actionnement étant désaccouplés l'un de l'autre par le mécanisme de désaccouplement après que le contenu de la seringue a été expulsé, de telle sorte que la seringue puisse être déplacée de sa position sortie à sa position rentrée par l'élément de rétraction.
